# EUROPEAN PATENT APPLICATION

(11) **EP 4 480 873 A1**
(43) Date of publication of application: **25.12.2024**
(21) Application number: 24181973.9
(22) Date of filing: 13.06.2024
(51) Int. Cl.: B65H 19/18

(54) **DEVICE AND RELATED APPARATUS FOR SPLICING A FIRST MATERIAL AND A SECOND MATERIAL FOR MAKING ABSORBENT ARTICLES**

(30) Priority: 22.06.2023 IT 202300012894
(71) Applicant: GDM S.P.A., 40133 Bologna (IT)
(72) Inventor: ZAVALLONI, Alessandro, BOLOGNA (IT); AIOLFI, Agostino, BOLOGNA (IT)
(74) Representative: Casadei, Barbara

(57) **Abstract**

A splicing device (8) comprises a head portion (9) provided with a first slot (10), configured for the passage of a first material (2), and with a second slot (11), configured for the passage of a second material (3);
the splicing device (8) comprises a first retaining member (19) for holding an edge (2a) of the first material (2), and a second retaining member (20) for holding an edge (3a) of the second material (3), each having a respective retaining surface (19a, 20a), configured to vary its orientation from a first configuration in which it is oriented towards the head portion (9) of the splicing device (8) to receive a respective edge (2a, 3a), to a second configuration in which the retaining surface (19a, 20a) is oriented in such a way as to position the respective edge (2a, 3a) of one of the two between the first material (2) and the second material (3) so it confronts the other, and vice versa.

## Description

This invention relates to a device and a related apparatus for splicing a first material and a second material used for making absorbent articles.

The production of disposable absorbent articles involves using flexible materials such as, by way of non-limiting example, materials like nonwoven fabric, elastic materials, adhesive tapes, polymer films, paper tapes and the like.

While the product is being formed, these materials are typically unwound from relatively large rolls and are used in a process in which the material is processed and generally combined with other materials.

In a production process, the use of rolls entails the presence of splicing devices whose purpose is to join a first material to a second material when the roll of one of the two materials is nearing depletion and needs to be replaced with a new roll.

During a splicing sequence, the material unwound from the new roll is spliced to the depleted material which is cut so that the material from the new roll can be used in the process.

To avoid interrupting the process, all the above steps must be carried out very rapidly and the materials to be spliced must be precisely aligned at the point of attachment.

In effect, misaligned splices can not only cause processing problems, accompanied by undesirable downtime but may also lead to the production of non-conformant products.

In prior art splicing devices, the operator manually positions the material to be spliced in a dedicated slot in the middle of the device.

However, owing to the variability of the materials to be spliced and the possibility that the material is not inserted into the slot properly, misalignment problems may occur and, as a result, splicing may be unsuccessful.

There is therefore a need to provide an improved splicing device which can ensure that the materials to be spliced are correctly aligned before and after a splicing sequence.

The splicing device comprises a head portion provided with a first slot, configured for the passage of a first material, and with a second slot, configured for the passage of a second material.

The splicing device comprises locking means and joining means selectively engageable with the first material and with the second material. The locking means and the joining means are configured to pass from a respective non-operating position to a respective operating position, and vice versa, where, at the respective operating position, the locking means are configured to hold the first material and the second material in place, and the joining means are configured to splice the first material to the second material.

The splicing device comprises a first cutting member, configured to proceed to cutting the first material, and a second cutting member, configured to proceed to cutting the second material.

The splicing device comprises a first retaining member for holding an edge of the first material, and a second retaining member for holding an edge of the second material, having a respective retaining surface, configured to vary its orientation from a first configuration in which it is oriented towards the head portion of the splicing device to receive a respective edge, to a second configuration in which the retaining surface is oriented in such a way as to position the respective edge of one of the two between the first material and the second material so it confronts the other, and vice versa. Preferably, the first retaining member and the second retaining member each have a respective retaining suction surface which is in fluid connection with suction means.

Preferably, a partializing element for partializing the retaining suction surface, interposed between it and the suction means, is configured to prevent suction through part of the retaining suction surface.

This allows the splicing device to handle different sizes.

Preferably, the first retaining member and the second retaining member each comprise a respective rotary member whose rotation about its axis causes the retaining surface to pass from the first configuration to the second configuration, and vice versa.

That way, the movement of the first retaining member and of the second retaining member can be controlled in a simple and effective manner.

Preferably, the locking means comprise a first body and a second body which are movable along a direction transverse to a feed direction of the first material and of the second material.

The first body and the second body are constrained to the movement of the rotary member of the respective first retaining member and second retaining member.

That is because the first body and the second body are disposed upstream of the first retaining member and of the second retaining member, with reference to the feeding of the first and second materials, hence without hindering insertion of the edge of the material on the respective retaining member.

Preferably, the splicing device comprises pneumatic actuating means to move the first retaining member and the second retaining member from the first configuration to the second configuration, and vice versa.

The Applicant has found that actuating in this way allows achieving speed and precision.

Preferably, the splicing device comprises elastic return means configured to keep the first retaining member and the second retaining member in the respective second configuration.

This ensures that the first retaining member and the second retaining member remain in the respective second configuration.

Preferably, the retaining surfaces of the first retaining member and of the second retaining member are at least partly covered with a spongy material of polymeric origin.

This facilitates splicing the first material and the second material to each other.

Preferably, the first retaining member and the second retaining member define the joining means.

In an embodiment, in which an adhesive substance is applied to the edge of the splicing material, the materials are spliced to each other by the first retaining member and the second retaining member.

Preferably, the splicing device comprise a frame having a first portion and second portion defining the aforementioned head portion. The first portion and the second portion are configured to pass from a closed position, where the first retaining member and the second retaining member are not accessible from the outside, to an open position, where the first retaining member and the second retaining member are accessible from the outside, and vice versa.

Preferably, a splicing apparatus which comprises the splicing device also comprises a first unwinding station to unwind a first roll of the first material, and a second unwinding station to unwind a second roll of the second material, and an accumulation zone where one between the first material and the second material exits the splicing device so that when the joining means are at the operating position, the first material and the second material are in a standby condition.

Further features and advantages of the invention are more apparent from the exemplary, hence non-limiting description which follows of a preferred embodiment of a splicing apparatus as illustrated in the accompanying drawings, in which:
- Figures 1 to 3 schematically illustrate a splicing device according to this invention in different steps in its operation;
- Figure 4 schematically illustrates a detail of the splicing device, with some parts cut away in order to better illustrate others;
- Figure 5 schematically illustrates an apparatus comprising the splicing device.

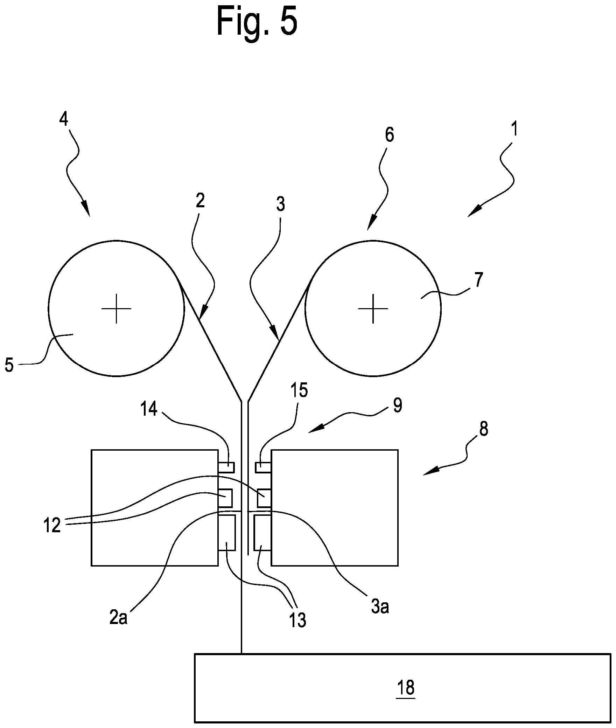

The reference numeral 1 denotes a splicing apparatus for splicing a first material 2 and a second material 3 used for making absorbent articles, as illustrated schematically in Figure 5.

The apparatus 1 comprises a first unwinding station 4 to unwind a first roll 5 of the first material 2, and a second unwinding station 6 to unwind a second roll 7 of the second material 3.

The apparatus 1 comprises a splicing device 8 which comprises a head portion 9 provided with a first slot 10, configured for the passage of a first material 2, fed by the first winding station 4, and with a second slot 11, configured for the passage of the second material 3, fed by the second unwinding station 6.

The first slot 10 and the second slot 11 are located at a central position of the splicing device 8.

From the first slot 10 and from the second slot 11, the first material 2 and the second material 3 advance parallel to each other along a substantially vertical feed direction.

It should be noted that the splicing device 8 is what is known as a "symmetrical" device, that is to say, one in which the same means/parts, are used, relative to a centre line, to handle the first material 2 and the second material 3 inserted therein, since the splicing device 8 is configured to splice the first material 2 to the second material 3 whether the depleted material is the first material 2 or the second material 3.

The splicing device 8 comprises locking means 12 and joining means 13 selectively engageable with the first material 2 and with the second material 3.

The locking means 12 and the joining means 13 are configured to pass from a respective non-operating position to a respective operating position, and vice versa. At the respective operating position, the locking means 12 are configured to hold the first material 2 and the second material 3 in place, and the joining means 13 are configured to splice the first material 2 to the second material 3.

The joining means 13 may be any means which are capable of joining the two materials to each other.

In an embodiment, for example, they may simply be in the form of a clamp or a gripper which joins the two materials. In such an embodiment, tape or glue may be applied to one of the two materials so that the two materials stick together when joined.

Alternatively, the joining means may be in the form of thermal welders or ultrasound welders.

The locking means 12 comprise a first body 16 and a second body 17 which are movable along a direction transverse to a feed direction of the first material 2 and of the second material 3.

Preferably, the first body 16 and the second body 17 are in the form of contoured plates.

The splicing device 8 comprises a first cutting member 14, configured to proceed to cutting the first material 2, and a second cutting member 15, configured to proceed to cutting the second material 3.

The first cutting member 14 or the second cutting member 15 is activated selectively when the first roll 5 of the first material 2 is depleted or the second roll 7 of the second material 3 is depleted, respectively, and after the joining means 13 have joined the first material 2 and the second material 3 to each other.

An accumulation zone 18 is provided where one between the first material 2 and the second material 3 exits the splicing device 8 so that when the joining means 13 are at the operating position, the first material 2 or the second material 3 is in a standby condition.

The accumulation zone 18 may include a first set of guide rollers, spaced from a second set of guide rollers.

The first set of guide rollers may be operatively associated with a carriage. The carriage may be movable towards the second set of guide rollers to release the material from the accumulation zone 18.

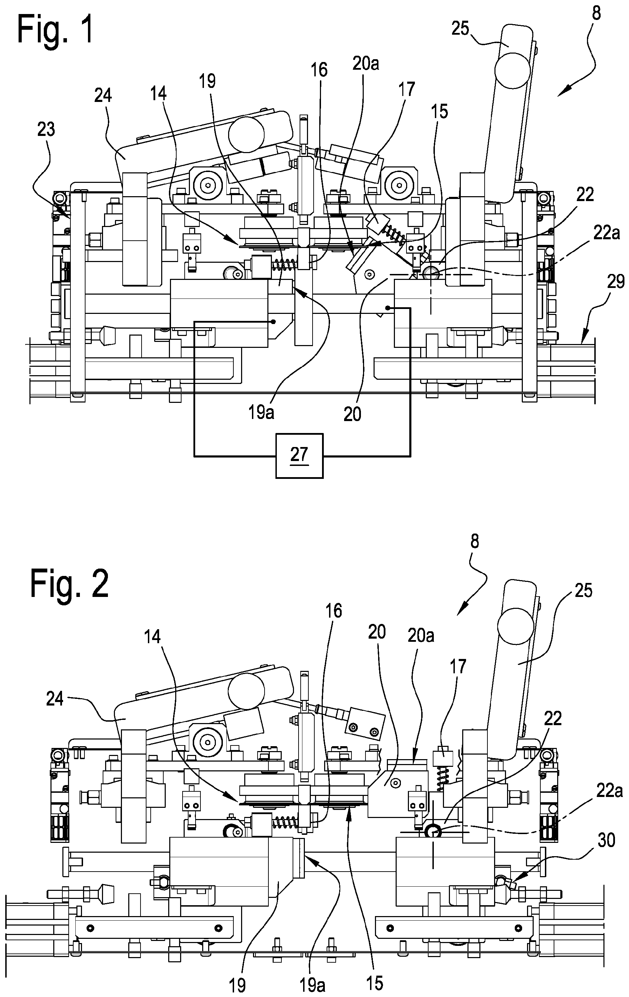

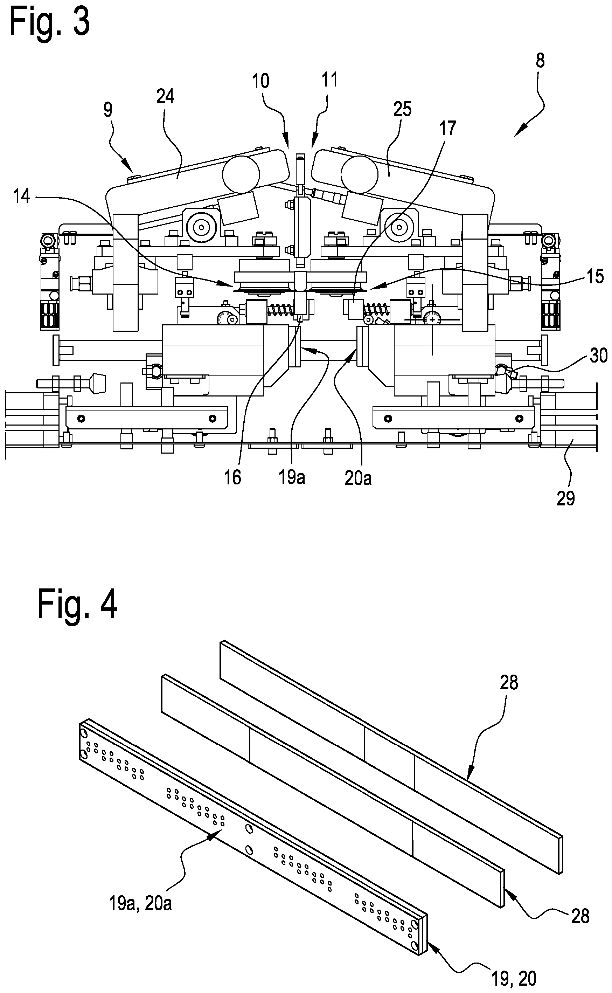

The splicing device 8 comprises a first retaining member 19 for holding an edge 2a of the first material 2, and a second retaining member 20 for holding an edge 3a of the second material 3, each having a respective retaining surface 19a, 20a, configured to vary its orientation from a first configuration in which it is oriented towards the head portion 9 of the splicing device 8 (schematically illustrated in Figure 2) to receive a respective edge 2a, 3a, to a second configuration in which the retaining surface 19a, 20a is oriented in such a way as to position the respective edge 2a, 3a of one of the two between the first material 2 and the second material 3 so it confronts the other (as illustrated schematically in Figure 3), and vice versa.

Advantageously, the presence of the first retaining member 19 and of the second retaining member 20, allows the operator to position the edge of the material to be spliced without misalignments since the retaining surfaces 19a, 20a of these members vary their orientation to position themselves so they are oriented towards the head portion 9 of the splicing device 8 so the operator can insert the edge and rest it on the other material so they can be spliced.

Preferably, the pneumatic actuating means 29 move the first retaining member 19 and the second retaining member 20 from the first configuration to the second configuration, and vice versa.

In order to ensure the passage from the first configuration to the second configuration, elastic return means 30 are provided which are configured to keep the first retaining member 19 and the second retaining member 20 in the respective second configuration.

The first retaining member 19 and the second retaining member 20 each have a respective retaining suction surface 19a, 20a which is in fluid connection with suction means 27.

A partializing element 28 for partializing the retaining suction surface 19a, 20a is interposed between it and the suction means 27.

The partializing element 28 is configured to prevent suction through part of the retaining suction surface 19a, 20a.

By way of example, Figure 4 schematically illustrates two partializing elements 28 which allow air to pass through a central portion whose size varies as a function of the size of the material to be processed.

This allows handling two or more sizes with the same splicing device 8 by varying the active suction portion of the retaining suction surface 19a, 20a. The first retaining member 19 and the second retaining member 20 each comprise a respective rotary member 22 whose rotation about its axis 22a causes the retaining surface 19a, 20a to pass from the first configuration to the second configuration, and vice versa.

The rotary member 22 has a range of angular movement between 0° and 90°.

The first body 16 and the second body 17 are constrained to the movement of the rotary member 22 of the respective first retaining member 19 and second retaining member 20.

Advantageously, in its second configuration, the retaining surface 19a, 20a is free of any encumbrance.

The first retaining member 19 and the second retaining member 20 each comprise a bar 21 which is provided with the respective retaining suction surface 19a, 20a.

By way of example, the rotary member 22 is in the form of one or more pins.

The splicing device 8 comprises a frame 23 having a first portion 24 and a second portion 25 defining said head portion 9.

The first portion 24 and the second portion 25 are configured to pass from a closed position, where the first retaining member 19 and the second retaining member 20 are not accessible from the outside, to an open position, where the first retaining member 19 and the second retaining member 20 are accessible from the outside, and vice versa.

By way of example, the open position of the second portion 25 is represented schematically in Figures 1 and 2. The closed position of the second portion 25 is represented schematically in Figure 3.

Advantageously, the interior of the splicing device 8 can be accessed by opening the first portion 24 and the second portion 25 of the frame 23 to allow the operator to correctly position the edge 2a, 3a of the first material 2 and the second material 3 on the respective retaining surface 19a, 20a. The first portion 24 and the second portion 25 are hinged to a respective fixed portion of the frame 23.

Optionally, the first portion 24 and the second portion 25 of the frame 23 each comprise a respective feed roller for feeding a respective first material 2 and second material 3.

The rollers each face a respective first slot 10 and second slot 11.

It should be borne in mind that if adhesive substances are used to join together the first material 2 and the second material 3, the first retaining member 19 and the second retaining member 20 define the joining means 13.

In order to optimize splicing between the first material 2 and the second material 3, the retaining surface 19a, 20a of the first retaining member 19 and of the second retaining member 20 is at least partly covered with a spongy material of polymeric origin. whose deformability is greater than that of metallic materials.

## Claims

1. A device for splicing a first material (2) and a second material (3), comprising a head portion (9) provided with a first slot (10), configured for the passage of the first material (2), and with a second slot (11), configured for the passage of the second material (3);
the splicing device (8) comprises locking means (12) and joining means (13) selectively engageable with the first material (2) and with the second material (3);
the locking means (12) and the joining means (13) are configured to pass from a respective non-operating position to a respective operating position, and vice versa, where, at the respective operating position, the locking means (12) are configured to hold the first material (2) and the second material (3) in place, and the joining means (13) are configured to splice the first material (2) to the second material (3);
the splicing device (8) comprises a first cutting member (14), configured to proceed to cutting the first material (2), and a second cutting member (15), configured to proceed to cutting the second material (3);
the splicing device (8) comprises a first retaining member (19) for holding an edge (2a) of the first material (2), and a second retaining member (20) for holding an edge (3a) of the second material (3), each having a respective retaining surface (19a, 20a), configured to vary its orientation from a first configuration in which it is oriented towards the head portion (9) of the splicing device (8) to receive a respective edge (2a, 3a), to a second configuration in which the retaining surface (19a, 20a) is oriented in such a way as to position the respective edge (2a, 3a) of one of the two between the first material (2) and the second material (3) so it confronts the other, and vice versa;
the first retaining member (19) and the second retaining member (20) each have a respective retaining suction surface (19a, 20a) which is in fluid connection with suction means (27);
said device comprises a partializing element (28) for partializing the retaining suction surface (19a, 20a) interposed between it and the suction means (27), the partializing element (28) being configured to prevent suction through part of the retaining suction surface (19a, 20a).

2. The device according to the preceding claim, wherein the first retaining member (19) and the second retaining member (20) each comprise a respective rotary member (22) whose rotation about its axis (22a) causes the retaining surface (19a, 20a) to pass from the first configuration to the second configuration, and vice versa.

3. The device according to the preceding claim, wherein the locking means (12) comprise a first body (16) and a second body (17) which are movable along a direction transverse to a feed direction of the first material (2) and of the second material (3), the first body (16) and the second body (17) being constrained to the movement of the rotary member (22) of the respective first retaining member (19) and second retaining member (20).

4. The device according to one or more of the preceding claims, comprising pneumatic actuating means (29) to move the first retaining member (19) and the second retaining member (20) from the first configuration to the second configuration, and vice versa.

5. The device according to one or more of the preceding claims, comprising elastic return means (30) configured to keep the first retaining member (19) and the second retaining member (20) in the respective second configuration.

6. The device according to one or more of the preceding claims, wherein the retaining surface (19a, 20a) of the first retaining member (19) and of the second retaining member (20) is at least partly covered with a spongy material of polymeric origin.

7. The device according to one or more of the preceding claims, wherein the first retaining member (19) and the second retaining member (20) define the joining means (13).

8. The device according to one or more of the preceding claims, wherein the splicing device (8) comprises a frame (23) having a first portion (24) and a second portion (25) defining said head portion (9); the first portion (24) and the second portion (25) are configured to pass from a closed position, where the first retaining member (19) and the second retaining member (20) are not accessible from the outside, to an open position, where the first retaining member (19) and the second retaining member (20) are accessible from the outside, and vice versa.

9. A splicing apparatus comprising the splicing device according to one or more of claims 1 to 8, comprising a first unwinding station (4) to unwind a first roll (5) of the first material (2), and a second unwinding station (6) to unwind a second roll (7) of the second material (3), and an accumulation zone (18) where one between the first material (2) and the second material (3) exits the splicing device (8) so that when the joining means (13) are at the operating position, the first material (2) or the second material (3) is in a standby condition.
